# FASCICULE DE BREVET EUROPEEN

(11) **EP 0 652 860 B1**
(45) Date de publication et mention de la délivrance du brevet: **24.09.1997**
(21) Numéro de dépôt: 94917695.2
(22) Date de dépôt: 01.06.1994
(51) Int. Cl.: C07C 43/13, C07C 323/25, A61K 7/48, A61K 7/50, A61K 7/06

(54) **UTILISATION DE COMPOSES HYDRO- ET FLUOROCARBONES DANS DES COMPOSITIONS COSMETIQUES, COMPOSES HYDRO- ET FLUOROCARBONES ET COMPOSITIONS COSMETIQUES EN COMPORTANT**
VERWENDUNG VON FLUORIERTEN KOHLENWASSERSTOFFDERIVATEN IN KOSMETISCHEN ZUSAMMENSETZUNGEN, FLUORIERTE KOHLENWASSERSTOFFDERIVATE, UND SIE ENTHALTENDE KOSMETISCHE ZUBEREITUNGEN
USE OF HYDROCARBON- AND FLUOROCARBON-CONTAINING COMPOUNDS, IN COSMETIC COMPOSITIONS, HYDROCARBON AND FLUOROCARBON-CONTAINING COMPOUNDS AND COSMETIC COMPOSITIONS COMPRISING SAME

(30) Priorité: 02.06.1993 FR 9306605
(43) Date de publication de la demande: 17.05.1995
(73) Titulaire: L'OREAL, 75008 Paris (FR)
(72) Inventeur: BOLLENS, Eric, F-94410 Saint-Maurice (FR); MAHIEU, Claude, F-75017 Paris (FR)
(74) Mandataire: Casalonga, Axel
(86) Numéro de dépôt international: FR9400634
(87) Numéro de publication internationale: WO9427944

(56) Documents cités:
- EP-A- 0 240 601
- EP-A- 0 457 688
- EP-A- 0 498 716
- WO-A-93/11103
- US-A- 4 113 748
- DATABASE WPI Week 8937, Derwent Publications Ltd., London, GB; AN 89-266844 & JP,A,1 193 236 (MITSUBISHI METAL KK) 3 Août 1989 cité dans la demande
- PATENT ABSTRACTS OF JAPAN vol. 17, no. 067 (C-1025) 10 Février 1993 & JP,A,04 275 248 (NEOS CO LTD) 30 Septembre 1992 cité dans la demande

## Description

La présente invention concerne l'utilisation de composés hydro- et fluorocarbonés dans des compositions cosmétiques et les compositions cosmétiques comportant ces composés, ainsi que certains composés nouveaux.

EP-A-498 716 et EP-A- 457 688 décrivent des compositions de lavage des matières kératiniques, en particulier des cheveux et/ou de la peau, comprenant dans un milieu aqueux, soit au moins une huile hydrocarbonée, (EP-A-'716), soit au moins une silicone (EP-A-'688), au moins un agent tensio-actif possédant des propriétés détergentes et au moins un alcool non-perfluoré ayant 27 à 44 atomes de carbone et comportant un ou deux groupements éther et/ou thioéther ou sulfoxyde.

Il est connu d'utiliser des perfluoropolyéthers, notamment dans des procédés de nettoyage, de protection, de maquillage de la peau ou bien de lavage des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation dans des compositions cosmétiques. Certains de ces composés, les perfluorométhylisopropyléthers, sont connus sous le nom de "FOMBLIN HC", commercialisés par la Société MONTEFLUO.

La demanderesse a maintenant découvert que certains composés présentent une solubilité améliorée par rapport aux FOMBLIN dans certains solvants usuels en cosmétique. Leurs propriétés leur permettent de pouvoir être facilement dispersés dans certains milieux cosmétiques tout en conservant une bonne stabilité aux compositions dans lesquelles ils interviennent.

Ainsi, la présente invention concerne l'utilisation de composés de formule :

R_{F}-(CH₂)ₙ-X-[C₃H₅(OH)]-Y-(CH₂)ₘ-R'_{F} (I)

dans laquelle C₃H₅(OH) représente les structures : 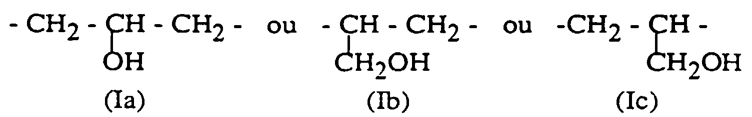
R_{F} et R_{F'}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques ou différents, sont O ou S.

Parmi ces composés, les composés préférés sont ceux pour lesquels R_{F} et R_{F'} désignent un radical alkyle perfluoré, linéaire ou ramifié en C₆-C₁₀, X et Y ne sont pas tous les deux O, n et m sont 2 et -C₃H₅(OH)- est linéaire.

Parmi les composés de formule (I), les composés de formule (I') suivante sont nouveaux :

R_{F}-(CH₂)ₙ-X-[C₃H₅(OH)]-Y-(CH₂)ₘ-R'_{F} (I')

dans laquelle C₃H₅(OH) représente les structures : 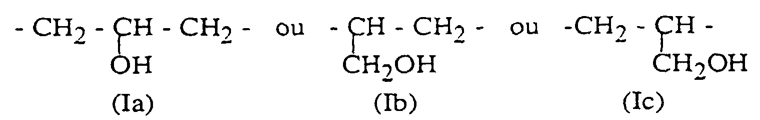
R_{F} et R_{F'}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O.

Les composés préférés de formule (I') sont ceux pour lesquels R_{F} et R_{F'} désignent un radical perfluoré, linéaire ou ramifié en C₆-C₁₀, n et m sont 2, et C₃H₅(OH) représente la structure (Ia).

Les composés de formule (I) selon l'invention peuvent être préparés en mettant en oeuvre la réaction d'un composé fluoré à hydrogène acide de formule (II) :

R_{F}-(CH₂)ₙ-X-H (II)

avec un époxyde de formule (III) : 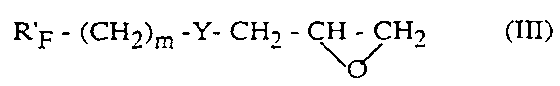 ou la réaction d'un composé fluoré à hydrogène acide de formule (IV) :

R_{F'}-(CH₂)ₘ-Y-H (IV)

avec un époxyde fluoré de formule (V) : 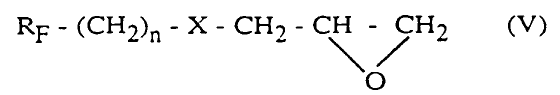 en présence d'un composé basique ou acide jouant le rôle de réactif ou de catalyseur, pour obtenir le composé de formule (I) correspondant, les substituants R_{F}, R_{H}, n et x ayant dans les formules (II) et (III), (IV) et (V), la même signification que dans la formule (I) et X et Y désignant O ou S.

Les composés de formule (V) sont décrits notamment dans le brevet US 3976698 ou dans les demandes de brevet EP 300358 et DE 2018461.

Lorsque X représente S dans la formule (II) ou (IV), on utilise de préférence un composé basique.

Les composés jouant le rôle de réactif ou de catalyseur peuvent donc être basiques, tels que les métaux alcalins, les hydroxydes de métaux alcalins ou alcalino-terreux, les alcoolates des métaux alcalins tels que les méthylates ou tertiobutylates, les hydrures alcalins tels que l'hydrure de sodium, les amines tertiaires telles que la pyridine ou la triéthylamine. Ils peuvent aussi être des bases de Lewis parmi lesquelles on peut citer les fluorures de césium, de rubidium, de potassium. Ces composés peuvent être également supportés sur un solide tel que l'alumine. De préférence, on utilise un alcoolate alcalin comme le méthylate de sodium ou une amine tertiaire comme la pyridine.

Ces composés peuvent également être acides, notamment lorsque le produit de départ de formule (II) ou (IV) est un alcool. De tels acides peuvent être les acides minéraux ou leurs sels avec des amines tertiaires ou encore des acides dits de Lewis tels que le trifluorure de bore, le tétrachlorure d'étain, le pentachlorure d'antimoine, utilisés seuls, en solution ou associés à un support usuel.

La concentration des composés acides ou basiques jouant le rôle de réactif ou de catalyseur et mis en oeuvre dans la réaction de préparation des composés de formule (I) peut être comprise entre 1 et 100% molaire, de préférence entre 2 et 10% molaire par rapport au composé fluoré à hydrogène acide de formule (II) ou (IV).

La réaction de préparation peut être mise en oeuvre en présence de solvant ou en l'absence de solvant.

Comme solvant, on peut utiliser des hydrocarbures aliphatiques tels que l'heptane, l'hexane ou des hydrocarbures cycliques tels que le cyclohexane, des hydrocarbures aromatiques tels que le toluène, des éthers tels que l'éther éthylique ou isopropylique ou le tert.-butylméthyléther, des éthers cycliques tels que le dioxanne, ou bien encore l'acétonitrile, le diméthylformamide, la N-méthylpyrrolidone, le diméthylacétamide.

Lorsque le composé de formule (II) ou (IV) est un thiol (X=S), les alcools tels que le méthanol, l'éthanol ou l'isopropanol peuvent aussi être utilisés comme solvant.

Pour préparer les composés de formule (I), on peut mélanger d'abord le composé à hydrogène acide de formule (II) ou (IV) avec le réactif ou catalyseur acide ou basique, sous atmosphère inerte. Pour réaliser le mélange, on peut opérer à une température comprise entre 20 et 180°C, de préférence entre 50 et 150°C. Par atmosphère inerte, on entend par exemple une atmosphère d'azote, d'argon ou d'hélium.

Le mélange peut être effectué, selon la nature du composé à hydrogène acide et du réactif ou catalyseur, en l'absence ou en présence de solvant.

On ajoute au mélange obtenu l'époxyde de formule (III) ou (V), cette addition pouvant s'effectuer en une seule fois ou progressivement, sur une période pouvant aller de 30 minutes à 2 heures par exemple.

Le temps de réaction est alors compris entre environ 1 heure et 24 heures, de préférence entre 1 heure et 3 heures.

Le composé issu de la réaction peut être oxydé lorsque celui-ci contient une fonction mercaptan, en sulfoxyde ou en sulfone, en présence d'eau oxygénée en milieu acide, selon des méthodes connues, notamment dans les demandes de brevet français FR 2 099 092 et FR 2 516 920.

Il peut être en outre nécessaire de neutraliser le mélange obtenu, et on peut séparer le composé synthétisé de façon usuelle, par distillation, par exemple.

Lorsque la réaction de préparation des composés de formule (I) est effectuée en présence de composé basique, elle conduit uniquement à des composés pour lesquels C₃H₅(OH) représente le groupement (Ia). Dans le cas où la réaction est effectuée en présence de composé acide, on peut obtenir un mélange de composés correspondant aux significations (Ia), (Ib) et (Ic) de C₃H₅(OH).

Les composés selon l'invention peuvent se présenter sous forme d'huile ou sous forme solide à la température ambiante.

Les composés de formule (I) où X et Y sont identiques, sont connus notamment par les demandes de brevet DE-2.702.607, JP 89-193236, JP 92-275248 et le brevet US-3.893.984.

De façon générale, ces composés utilisés dans des compositions cosmétiques permettent d'en améliorer les propriétés cosmétiques. Ils confèrent de la douceur, de la brillance et un toucher non collant aux matières kératiniques telles que la peau, les cheveux et les ongles. Par ailleurs, certains de ces composés se présentant sous forme d'huile incolore, ils peuvent permettre d'obtenir des émulsions transparentes.

C'est pourquoi la présente invention concerne également des compositions cosmétiques caractérisées en ce qu'elles comportent au moins un composé de formule (I) définie ci-dessus et au moins un adjuvant cosmétique.

Ces compositions peuvent se présenter sous forme d'émulsions, de laits ou de crèmes, de lotions huileuses ou oléoalcooliques, sous forme de gels gras ou oléoalcooliques, de dispersions vésiculaires à base de lipides amphiphiles ioniques ou non-ioniques, de bâtonnets solides, de pâte, de spray ou de mousse aérosol.

Selon la forme des compositions auxquelles sont incorporés les composés de l'invention, ces compositions comportent par ailleurs les adjuvants et additifs usuels pour la forme choisie.

Plus précisément, ces compositions peuvent être des laits et des crèmes pour les soins de la peau ou des cheveux, des crèmes, lotions ou laits démaquillants, des crèmes, gels, laits ou lotions anti-solaire, des crèmes ou mousses de rasage, des lotions après-rasage, des shampooings ou des après-shampooings, des gels de coiffage, de compositions de coloration capillaire, de compositions pour la déformation permanente des cheveux, des déodorants corporels, des pâtes dentifrices, des laques, des produits de soin des lèvres ou des ongles.

Ces compositions cosmétiques peuvent être également utilisées comme produit de maquillage des cils, des sourcils, des ongles, des lèvres ou de la peau comme les crèmes de traitement de l'épiderme, des fonds de teint, des bâtons de rouge à lèvres, des fards à paupières ou à joues, des eye-liners ou mascara, des vernis à ongles, par exemple.

Selon l'invention, les composés de formule (I) représentent de 0,1 à 25%, et de préférence de 0,1 à 15% du poids total de la composition.

Parmi les adjuvants cosmétiques usuels pour ce genre de composition, peuvent être présents en outre dans les compositions selon l'invention, des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des agents réducteurs, des antioxydants et des anti-radicaux libres.

Plus précisément, comme corps gras, on peut utiliser une huile ou une cire ou leur mélange, des acides gras, des alcools gras, des esters d'acides gras tels que les triglycérides d'acides gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et des gommes de silicone et les isoparaffines.

Parmi les cires animales, fossiles, végétales, minérales ou de synthèse, on peut notamment citer les cires d'abeille, de Caroube, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, l'alcool isopropylique, le propylèneglycol, le glycérol, le sorbitol, les cétones telles que l'acétone, les esters tels que l'acétate de butyle ou l'acétate d'éthyle, le myristate d'isopropyle, le toluène, par exemple.

A titre d'agent épaississant, on peut citer les dérivés de cellulose, les dérivés d'acide polyacrylique, les gommes de guar ou de caroube, ainsi que la gomme de xanthane, par exemple.

Parmi les tensio-actifs, on peut citer notamment les tensio-actifs non-ioniques tels que les alkyl(C₈-C₂₄)polyglycosides où le nombre de motif glucoside est compris entre 1 et 15 et les tensio-actifs non-ioniques du type polyglycérolé.

Les alkylpolyglycosides sont notamment les produits vendus sous la dénomination APG, tels que les produits APG 300, APG 350, APG 500, APG 550, APG 625, APG base 10-12; les produits vendus par la Société SEPPIC sous les dénominations TRITON CG 110 et TRITON CG 312.

Les composés polyglycérolés sont les dérivés qui résultent de la condensation de 1 à 10, de préférence de 2 à 6 moles de glycidol par mole d'alcool ou d'alphadiol en C₁₀-C₁₄, de diglycolamide d'acides gras en C₁₂-C₁₈, tels que décrits dans les brevets FR 1 477 048, 2 328 763, 2 091 516, 2 169 787.

Les dispersions vésiculaires de lipides amphiphiles ioniques ou non-ioniques citées ci-dessus, peuvent être préparées selon des procédés usuels dont on trouve une liste non limitative dans "Les liposomes en biologie cellulaire et pharmacologie" Edition INSERM/John Libbery Eurotext, (1987), p. 6 à 18.

Pour les compositions sous forme de pâte dentifrice, on peut utiliser les adjuvants et additifs usuels comme des agents de polissage tels que la silice, des agents actifs comme les fluorures tels que le fluorure de sodium, et éventuellement des agents édulcorants tels que le saccharinate de sodium.

Les exemples suivants sont destinés à illustrer l'invention et ne sauraient être considérés comme limitatifs de sa portée.

### EXEMPLES DE PREPARATION

### EXEMPLE I

### 1-(2'-F-hexyl-éthylthio) 3-(2'-F-hexyl-éthoxy)-2-propanol

A la température de 25°C, sous agitation et sous courant d'azote, on ajoute 1,33 g d'une solution méthanolique de méthylate de sodium (environ 30% - 5,65 meq g⁻¹) à 57 g de 2-F-hexyl-éthanethiol, en une minute.

Le mélange est chauffé à 70°C. On évapore sous vide le méthanol présent dans le milieu.

Le 2-F-hexyl-éthylglycidyléther (63 g-0,15 mole) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 7,5 ml de HCl normal.

Le 1-(2'-F-hexyl-éthylthio)3-(2'-F-hexyl-éthoxy)-2-propanol est séparé par distillation : Eb = 170°C/133 Pa.

On obtient 85 g (71%) d'une huile translucide incolore.

| Analyse élémentaire : | | | | |
|---|---|---|---|---|
| | %C | %H | %S | %F |
| Calculé | 28,51 | 1,76 | 4,01 | 61,72 |
| | | | | |
| Mesuré | 28,60 | 1,79 | 4,32 | 61,54 |

### EXEMPLE II

### 1,3-bis-(2'-F-hexyl-éthylthio)-2-propanol

Dans un tricol de 1 litre, on introduit 14,7 g de méthylate de sodium anhydre en poudre que l'on recouvre de 300 ml de méthanol anhydre également. On solubilise le méthylate dans le méthanol sous atmosphère d'azote et à la température ambiante en 10 minutes.

On ajoute, en 10 minutes, 99,75 g de 2-F-hexyl-éthanethiol (0,2625 mole) à la température ambiante; puis, le mélange étant refroidi à environ 5°C par un bain de glace, on ajoute une solution de 15 g de 1,3-dichloro-2-propanol (0,131 mole) dans 75 ml de méthanol.

Lorque l'addition est terminée, on retire le bain de glace et on laisse le mélange revenir à la température ambiante. On chauffe alors 1 heure à 40°C.

Le mélange est hydrolysé par 500 ml d'eau, puis le produit est extrait à l'éther isopropylique, la phase organique est séchée sur sulfate de sodium, filtrée sur papier filtre, puis le solvant est évaporé sous pression réduite.

On obtient une huile très pâle qui se solidifie en refroidissant.

Le produit est repris dans 400 ml d'éther de pétrole, trituré, puis filtré et séché sur papier filtre.

On obtient 65 g (62%) de 1,3-bis-(2'-F-hexyl-éthylthio)-2-propanol sous la forme d'un solide blanc dont le point de fusion est de 49°C.

| Analyse élémentaire : | | | | |
|---|---|---|---|---|
| | %C | %H | %S | %F |
| Calculé | 27,95 | 1,73 | 7,85 | 60,51 |
| | | | | |
| Mesuré | 27,85 | 1,63 | 7,81 | 60,38 |

### EXEMPLE III

### 1,3-bis-(2'-F-octyl-éthylthio)-2-propanol

Dans un tricol de 1 litre, on introduit 14,7 g de méthylate de sodium anhydre en poudre que l'on recouvre de 300 ml de méthanol anhydre également. On solubilise le méthylate dans le méthanol sous atmosphère d'azote et à la température ambiante en 10 minutes.

On ajoute, en 15 minutes, 125 g de 2-F-octyl-éthanethiol (0,2625 mole) à 25°C; puis, le mélange étant refroidi entre 0 et 5°C, on ajoute une solution de 15 g de 1,3-dichloro-2-propanol (0,131 mole) dans 80 ml de méthanol, en 10 minutes.

Après retour à la température ambiante, le mélange est chauffé à 40°C pendant 1 heure.

Le mélange est alors hydrolysé par 500 ml d'eau, puis le mélange est extrait par 600 ml d'éther isopropylique.

La phase organique est décantée, séchée sur sulfate de sodium, filtrée sur papier filtre, puis le solvant est évaporé.

On obtient un solide qui est repris dans 500 ml d'éther de pétrole, trituré, puis filtré sur verre fritté n°4.

On obtient finalement 102 g (77%) de 1,3-bis-(2'-F-octyl-éthylthio)-2-propanol sous la forme d'une poudre blanche dont le point de fusion est de 91°C.

| Analyse élémentaire : | | | | |
|---|---|---|---|---|
| | %C | %H | %S | %F |
| Calculé | 27,18 | 1,39 | 6,31 | 63,55 |
| | | | | |
| Mesuré | 27,22 | 1,47 | 6,25 | 63,87 |

### EXEMPLES DE FORMULATION

### EXEMPLE 1

### Emulsion huile-dans-eau anti-solaire

### Phase A

- Mélange d'alcool cétylstéarylique et d'alcool cétylstéarylique oxyéthyléné à 33 moles d'oxyde d'éthylène (80/20), vendu sous la dénomination "DEHSCONET 390" par la Société TENSIA 7 g
- Stéarate de glycéryle vendu sous la dénomination "GELEOL COPEAUX" par la Société GATTEFOSSE 2 g
- Huile de silicone vendue sous la dénomination "SILBIONE HUILE 70 047 V 300" par la Société RHONE POULENC 1,5 g
- Alcool cétylique 1,5 g
- Huile de vaseline 15 g
- Composé de l'exemple (I) 0,5 g
- Glycérol 20 g
- Conservateurs qs

### Phase B

- 2-éthylhexylparaméthoxycinnamate, vendu sous la dénomination "PARSOL MCX" par la Société GIVAUDAN 5 g
- Eau déminéralisée stérilisée qsp 100 g

On réalise l'émulsion en additionnant la phase grasse A portée aux environs de 80°C à la phase aqueuse B portée à la même température et sous agitation rapide. On obtient une émulsion huile-dans-eau se présentant sous la forme d'une crème.

### EXEMPLE 2

### Emulsion huile-dans-eau autobronzante

### Phase A

- Mélange d'alcool cétylstéarylique et d'alcool cétylstéarylique oxyéthyléné à 33 moles d'oxyde d'éthylène (80/20), vendu sous la dénomination "DEHSCONET 390" par la Société TENSIA 7 g
- Stéarate de glycéryle vendu sous la dénomination "GELEOL COPEAUX" par la Société GATTEFOSSE 2 g
- Huile de silicone vendue sous la dénomination "SILBIONE HUILE 70 047 V 300" par la Société RHONE POULENC 1,5 g
- Alcool cétylique 1,5 g
- Huile de vaseline 15 g
- Composé de l'exemple (II) 0,3 g
- Glycérol 20 g
- Conservateurs qs

### Phase B

- Dihydroxyacétone 3 g
- Eau déminéralisée stérilisée qsp 100 g

Le mode opératoire est identique à celui de l'exemple 1.

### EXEMPLE 3

### Emulsion huile-dans-eau soin après-soleil

### Phase A

- Mélange d'alcool cétylstéarylique et d'alcool cétylstéarylique oxyéthyléné à 33 moles d'oxyde d'éthylène (80/20), vendu sous la dénomination "DEHSCONET 390" par la Société TENSIA 7 g
- Stéarate de glycéryle vendu sous la dénomination "GELEOL COPEAUX" par la Société GATTEFOSSE 2 g
- Huile de silicone vendue sous la dénomination "SILBIONE HUILE 70 047 V 300" par la Société RHONE POULENC 1,5 g
- Alcool cétylique 1,5 g
- Huile de vaseline 15 g
- Composé de l'exemple (III) 0,2 g
- Glycérol 20 g
- Conservateurs qs

### Phase B

- α-bisabolol 0,5 g
- Eau déminéralisée stérilisée qsp 100 g

Le mode opératoire est identique à celui de l'exemple 1.

### EXEMPLE 4

### Crème de soin - Emulsion huile-dans-eau

On prépare une crème de soin sous forme d'émulsion huile-dans-eau, ayant la composition suivante :

### Phase A

- Tensio-actif non-ionique de type hydroxypropyléther obtenu par condensation, en catalyse alcaline, de 3,5 moles de glycidol sur un mélange d'alphadiols en C₁₁-C₁₄ selon le brevet français n° 2.091.516 2,4 g
- Parahydroxybenzoate de méthyle 0,2 g
- Glycérine 5 g
- Eau 22,06 g

### Phase A'

- Gel de polyacrylate de glycéryle dans l'eau (50/50), vendu sous la dénomination "HISPAGEL 100" par la Société HISPANO CHEMICA 56,14 g

### Phase B

- Alcool cétylique 1 g
- Composé de l'exemple I 5 g
- Huile amandes d'abricots 5 g
- Huile de sésame 1,5 g
- Triglycéride d'acide caprylique/caprique, vendu sous la dénomination "MIGLYOL 812" par la Société HULS 1,5 g
- Parahydroxybenzoate de propyle 0,2 g

### Mode opératoire :

On chauffe la phase A à 80°C. On ajoute, sous agitation, la phase A', puis la phase B préalablement chauffée à 80°C. On laisse refroidir à température ambiante tout en maintenant l'agitation.

Le composé de l'exemple I peut être remplacé par le composé de l'exemple II ou de l'exemple III.

### EXEMPLE 5

### Rouge à lèvres

On prépare un bâton de rouge à lèvres ayant la composition suivante :
- Composé de l'exemple I 1 g
- Ozokérite 14,90 g
- Cire microcristalline 4,90 g
- Cire de Candellila 7,40 g
- Huile de Jojoba 6,20 g
- Huile de ricin 1,20 g
- Lanoline liquide 18,60 g
- Lanoline acétylée 9,90 g
- Huile de vaseline 11,10 g
- Talc 3,70 g
- Micatitane 8,70 g
- D & C Red n° 7 Ca lake 5,20 g
- D & C Red n° 7 Ba lake 2,80 g
- FD & C Yellow n° 5 1 g
- Dioxyde de titane 3,10 g
- Butylhydroxytoluène 0,30 g
- Parfum qs
en mélangeant les huiles à une température de 50 à 60°C. Les pigments et laques organiques sont broyés dans la phase huileuse.

On ajoute alors les cires fondues, le talc et le micatitane, puis le parfum.

La composition est alors coulée dans un moule.

L'application du rouge à lèvres est aisée (glisse facilement) et celui-ci confère de la douceur aux lèvres.

Le composé de l'exemple I peut être remplacé par le composé de l'exemple II ou de l'exemple III.

### EXEMPLE 6

### Vernis à ongles

On prépare un vernis à ongles ayant la composition suivante :
- Composé de l'exemple I 1 g
- Nitrocellulose 10,75 g
- Résine toluène sulfonamide formaldéhyde, vendue sous la dénomination "KETJENFLEX MS80" par la Société AKZO 9,70 g
- Acétylcitrate de tributyle vendu sous la dénomination "CITROFLEX A4" par la Société PFIZER 6,45 g
- Toluène 30,70 g
- Acétate de butyle 21,50 g
- Acétate d'éthyle 9,20 g
- Alcool isopropylique 7,70 g
- Acide citrique 0,05 g
- Stéaralkonium hectorite 1,45 g
- Pigments 1,50 g

L'étalement du vernis sur l'ongle est aisé et on obtient un film présentant une bonne adhésion sur l'ongle ainsi qu'une bonne brillance. On observe également une bonne tenue de la brillance dans le temps.

### EXEMPLE 7

### Shampooing

- Lauryléther sulfate de sodium (C₁₂-C₁₄ 70/30) oxyéthyléné à 2,2 moles d'oxyde d'éthylène en solution aqueuse à 28%, vendu sous la dénomination "EMPICOL ESB/3 FL" par la Société MARCHON 8,4 g MA
- N-cocoamido éthyl N-éthoxycarboxyméthyl glycinate 4 g MA
- Hydroxyéthylcellulose réticulé à l'épichlorhydrine quatemisé par la triméthylamine, vendu sous la dénomination "CELQUAT SC 240" par la Société NATIONAL STARCH 0,35 g
- Distéarate de glycol (C₁₆/C₁₈ 30/70) 2 g
- Monoisopropanolamide d'acide de coprah 3 g
- Acide polyacrylique réticulé vendu sous la dénomination "CARBOPOL 980" par la Société GOODRICH 0,2 g
- Composé de l'exemple I 2 g
- Conservateurs, parfum
- Eau qsp 100 g

Le pH est ajusté à 7,5 par de l'hydroxyde de sodium.

### EXEMPLE 8

### Gel de coffrage

- Acide polyacrylique réticulé vendu sous la dénomination "CARBOPOL 980" par la Société GOODRICH 0,5 g
- Copolymère vinylpyrrolidone/acétate de vinyle (65/35), vendu sous la dénomination "LUVISCOL VA 64" par la Société BASF 2 g
- Composé de l'exemple I 0,5 g
- Eau qsp 100 g

### EXEMPLE 9

### Composition de permanente

On prépare une composition réductrice de déformation permanente des cheveux en procédant au mélange des ingrédients suivants :
- Acide thioglycolique 7 g
- Thioglycolate de glycérol à 68% de MA dans le glycérol 2 g MA
- Alcool oléique oxyéthyléné à 20 moles d'oxyde d'éthylène, vendu sous la dénomination "BRIJ 98" par la Société ICI 2 g
- Composé de l'exemple I 0,3 g
- Monoéthanolamine qs pH=7,5
- Eau déminéralisée qsp 100 g

Cette composition est appliquée sur des cheveux mouillés préalablement enroulés sur des rouleaux. Après avoir laissé agir la composition pendant 15 minutes, on rince abondamment à l'eau, puis on applique la composition oxydante suivante :
- Eau oxygénée qs 8 volumes
- Alcool oléique oxyéthyléné à 20 moles d'oxyde d'éthylène, vendu sous la dénomination "BRIJ 98" par la Société ICI 1 g
- Composé de l'exemple I 0,1 g
- Acide phosphorique qs pH=3,0
- Eau déminéralisée qsp 100 g

On laisse agir la composition pendant 5 minutes, puis on enlève les rouleaux et on rince abondamment la chevelure à l'eau. Après séchage sous casque, les cheveux présentent de belles boucles.

### EXEMPLE 10

On prépare une pâte dentifrice en mélangeant les composés suivants :
- Composé de l'exemple I 0,25 g
- Silice précipitée amorphe vendue sous la dénomination "TIXOSIL 73" par la Société RHONE POULENC 12 g
- Silice précipitée amorphe vendue sous la dénomination "TIXOSIL 333" par la Société RHONE POULENC 8 g
- Carboxyméthylcellulose de sodium vendu sous la dénomination "BLANOSE 9M 31 F" par la Société HERCULES 1,4 g
- Sorbitol en solution aqueuse à 70% de MA 22,4 g MA
- Laurylsulfate de sodium en poudre, vendu sous la dénomination "EMPICOL LZV/E" par la Société MARCHON à 93% de MA 1,67 g MA
- Monofluorophosphate de sodium 0,76 g
- Dioxyde de titane 0,6 g
- Parahydroxybenzoate de méthyle 0,2 g
- Saccharinate de sodium 0,15 g
- Arôme qs
- Eau qsp 100 G

## Revendications

1. Utilisation de composés de formule (I) :
R_{F}-(CH₂)ₙ-X-[C₃H₅(OH)]-Y-(CH₂)ₘ-R'_{F} (I)
dans laquelle C₃H₅(OH) représente les structures : 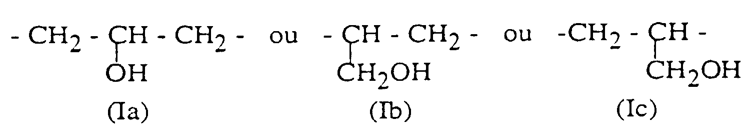
R_{F} et R_{F'}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques ou différents, sont O ou S,
dans des compositions cosmétiques.

2. Utilisation selon la revendication 1, caractérisée en ce que, dans la formule (I), R_{F} et R'_{F} représentent un radical alkyle perfluoré, linéaire ou ramifié en C₆-C₁₀;
X et Y ne sont pas tous les deux O;
n et m sont 2, et -C₃H₅(OH)- est

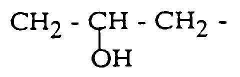

3. Composition cosmétique comportant au moins un composé de formule (I) :
R_{F}-(CH₂)ₙ-X-[C₃H₅(OH)]-Y-(CH₂)ₘ-R'_{F} (I)
dans laquelle C₃H₅(OH) représente les structures : 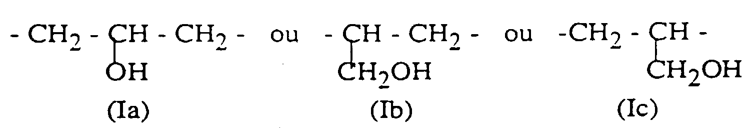
R_{F} et R_{F'}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques ou différents, sont O ou S,
et au moins un adjuvant cosmétique.

4. Composition cosmétique selon la revendication 3, caractérisée en ce qu'elle comporte un ou plusieurs adjuvants choisis dans le groupe formé des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des agents réducteurs, des antioxydants et des anti-radicaux libres.

5. Composition cosmétique selon la revendication 3 ou 4, caractérisée en ce qu'elle comporte 0,1 à 25% en poids du composé de formule (I).

6. Composition cosmétique selon l'une des revendications 3 à 5, caractérisée en ce qu'elle est sous forme de lait, crème, lotion huileuse ou oléo-alcoolique, gel gras ou oléo-alcoolique, dispersion vésiculaire à base de lipides amphiphiles ioniques ou non-ioniques, bâtonnet solide, pâte, spray ou mousse aérosol.

7. Composition cosmétique selon les revendications 3 à 6, caractérisée en ce qu'elle est sous forme de lait, de crème de soin pour la peau ou les cheveux, de crème, lotion ou lait démaquillant, de crème, gel, lotion ou lait anti-solaire, de crème ou mousse de rasage, de lotion après-rasage, de shampooing ou d'après-shampooing, de déodorant corporel, de pâte dentifrice, de laque, de produit de maquillage des cils, sourcils, ongles, lèvres ou peau, de crème de traitement de l'épiderme, de fond de teint, de bâton de rouge à lèvres, de fard à paupières ou à joues, d'eye-liner, mascara ou de produit de soin pour les lèvres ou les ongles.

8. Composés de formule (I'):
R_{F}-(CH₂)ₙ-X-[C₃H₅(OH)]-Y-(CH₂)ₘ-R'_{F} (I')
dans laquelle C₃H₅(OH) représente les structures : 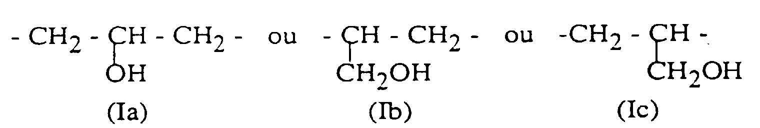
R_{F} et R_{F'}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O.

## Patentansprüche

1. Verwendung von Verbindungen der Formel (I)
R_{F}-(CH₂)ₙ-X-[C₃H₅(OH)]-Y-(CH₂)ₘ-R'_{F} (I)
worin
worin C₃H₅(OH) die Strukturen: 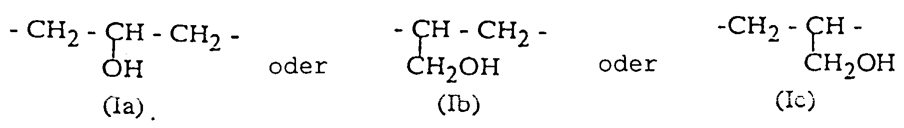
R_{F} und R'_{F}, gleich oder verschieden, einen linearen oder verzweigten C₄₋₂₀-Perfluoralkylrest oder eine Mischung linearer oder verzweigter C₄₋₂₀-Perfluoralkylreste und m und n, gleich oder verschieden, 0, 1, 2, 3 oder 4 darstellen,
und worin X und Y, gleich oder verschieden, 0 oder S sind, in kosmetischen Zusammensetzungen.

2. Verwendung gemäß Anspruch 1,
dadurch **gekennzeichnet,** daß
in der Formel (I) R_{F} und R'_{F} einen linearen oder verzweigten C₆₋₁₀-Perfluoralkylrest darstellen,
X und Y alle beide nicht 0 sind und
n und m 2 betragen und -C₃H₅(OH)- -CH₂-CH-OH-CH₂- ist.

3. Kosmetische Zusammensetzung, enthaltend mindestens eine Verbindung der Formel (I):
R_{F}(CH₂)ₙ-X-[C₃H₅(OH)]-Y-(CH₂)ₘ-R'_{F} (I)
worin C₃H₅(OH) die Strukturen: 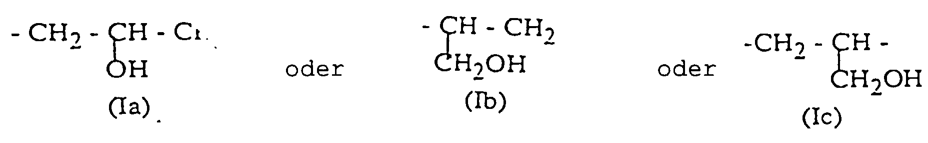 R_{F} und R'_{F}, gleich oder verschieden, einen linearen oder verzweigten C₄₋₂₀-Perfluoralkylrest oder eine Mischung linearer oder verzweigter C₄₋₂₀-Perfluoralkylreste und m und n, gleich oder verschieden, 0, 1, 2, 3 oder 4 darstellen und X oder Y, gleich oder verschieden, O oder S sind,
sowie mindestens einen kosmetischen Hilfsstoff.

4. Kosmetische Zusammensetzung gemäß Anspruch 3,
dadurch **gekennzeichnet,** daß
sie einen oder mehrere Hilfsstoffe enthält, ausgewählt aus der Gruppe aus üblichen Fettkörpern, organischen Lösungsmitteln, Silikonen, Verdickungsmitteln, Weichmachern, Sonnenfilterstoffen für UV-A oder UV-B oder für den langwelligen Bereich, aus Antischaummitteln, Hydratisiermitteln, Feuchtigkeitsmitteln, Parfüm-Produkten, Konservierungsstoffen, oberflächenaktiven Mitteln, Beaufschlagungsmitteln, Sequestriermitteln, Emulgatoren, anionischen, kationischen, nicht-ionischen und amphoteren Polymeren oder deren Mischungen, Antischweißmitteln, alkalilsch stellenden Mitteln, Färbemitteln, Pigmenten, Treibmitteln, reduzierenden Mitteln, Antioxidantien und aus Abfangmitteln für freie Radikale.

5. Kosmetische Zusammensetzung gemäß Anspruch 3 oder 4,
dadurch **gekennzeichnet,** daß
sie 0,1 bis 25 Gew.% Verbindung der Formel (I) enthält.

6. Kosmetische Zusammensetzung gemäß einem der Ansprüche 3 bis 5,
dadurch **gekennzeichnet,** daß
sie in Form einer Milch, Creme, ölartigen oder oleoalkoholischen Lotion, eines fetten oder oleoalkoholischen Gels, einer bläschenartigen Dispersion auf Basis amphiphiler ionischer oder nicht-ionischer Lipide, eines Feststoffstäbchens, einer Paste, eines Spray oder eines Aerosol-Schaums vorliegt.

7. Kosmetische Zusammensetzung gemäß einem der Ansprüche 3 bis 6,
dadurch **gekennzeichnet,** daß
sie in Form einer Milch, Pflegecreme für die Haut oder Haare, Creme, Lotion, Milch zum Entschminken, einer Creme, eines Gels, einer Lotion oder Milch als Sonnenschutzmittel, einer Creme oder eines Schaums zur Rasur, einer Nach-Rasur-Lotion, eines Shampoo, Nach-Shampoo, Körperdeodorant, einer Zahnpaste, eines Lacks, eines Produkts zum Schminken der Wimpern, Augenbrauen, Nägel, Lippen oder der Haut, einer Creme zur Behandlung der Epidermis, einer Teint-Grundlage, eines Lippenstifts, einer Schminke für Augenlider oder Wanken, eines Augenlinierstifts, einer Wimperntusche oder eines Produkts zur Pflege der Lippen oder Nägel vorliegt.

8. Verbindungen der Formel (I'):
R_{F}-(CH₂)ₙ-X-[C₃H₅(OH)]-Y-(CH₂)ₘ-R'_{F} (I')
worin C₃H₅(OH) die Strukturen: 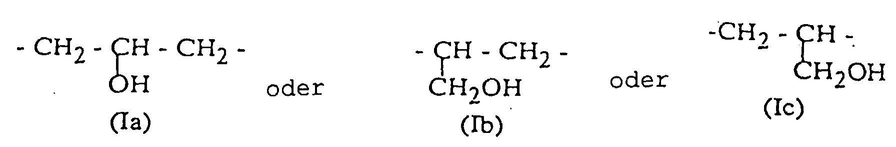
R_{F} und R'_{F}, gleich oder verschieden, einen linearen oder verzweigten C₄₋₂₀-Perfluoralkylrest oder eine Mischung linearer oder verzweigter C₄₋₂₀-Pertiuoralkylreste und
m und n, gleich oder verschieden, 0, 1, 2, 3 oder 4 darstellen und
X O und Y S oder X S und Y O sind.

## Claims

1. Use in a cosmetic composition of a compound with formula (I):
R_{F}-(CH₂)ₙ-X-[C₃H₅(OH)]-Y-(CH₂)ₘ- R'_{F} (I)
where
C₃H₅(OH) represents the structures: 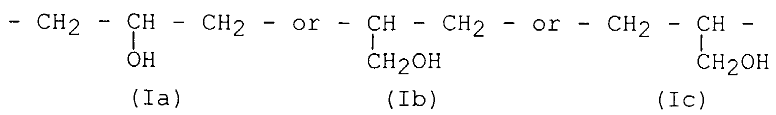
R_{F} and R'_{F}, which may be identical or different, represent a perfluorinated linear or branched C₄-C₂₀ alkyl radical or a mixture of perfluorinated linear or branched C₄-C₂₀ alkyl radicals;
m and n, which may be identical or different, are 0, 1, 2, 3 or 4;
X and Y, which may be identical or different, represent O or S.

2. Use according to claim 1 characterised in that in formula (I) R_{F} and R'_{F} represent a linear or branched perfluorinated C₆-C₁₀ alkyl radical;
X and Y are not both O;
n and m equal 2 and -C₃H₅(OH)- is

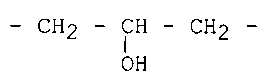

3. A cosmetic composition containing at least one compound with formula (I):
R_{F}-(CH₂)ₙ-X-[C₃H₅(OH)]-Y-(CH₂)ₘ-R'_{F} (I)
where
C₃H₅(OH) represents the structures: 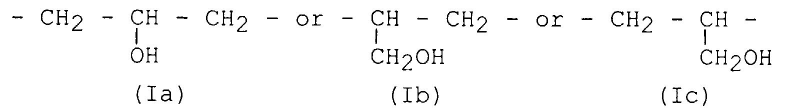
R_{F} and R'_{F}, which may be identical or different, represent a perfluorinated linear or branched C₄-C₂₀ alkyl radical or a mixture of perfluorinated linear or branched C₄-C₂₀ alkyl radicals;
m and n, which may be identical or different, are 0, 1, 2, 3 or 4;
X and Y, which may be identical or different, represent O or S,
and at least one cosmetic additive.

4. A cosmetic composition according to claim 3 characterised in that it contains one or more additives selected from the group formed by the usual fatty substances, organic solvents, silicones, thickeners, softeners, UV-A or UV-B or broad spectrum solar filters, anti-foaming agents, moisturising agents, humectants, fragrances, preservatives, surfactants, fillers, sequestrating agents, emulsifiers, anionic, cationic, non-ionic or amphoteric polymers and their mixtures, antiperspirants, alkalinising agents, dyes, pigments, propellants, reducing agents, anti-oxidising agents and free radical absorbers.

5. A cosmetic composition according to claim 3 or claim 4 characterised in that it contains 0.1% to 25% by weight of the compound with formula (I).

6. A cosmetic composition according to any one of claims 3 to 5 characterised in that it is in the form of a milk or cream, oily or oleoalcoholic lotion, oily or oleoalcoholic gel, ionic or non-ionic amphiphilic lipid based vesicular dispersion, solid stick, paste, spray or aerosol foam.

7. A cosmetic composition according to any one of claims 3 to 6 characterised in that it is in the form of a milk or cream for skin or hair, a make-up removing cream, lotion or milk, a sun protection cream, gel, milk or lotion, a shaving cream or foam, an aftershave lotion, a shampoo or conditioner, a body deodorant, a toothpaste, a lacquer, an eyelid, eyelash, nail, lip or skin make-up, a skin treatment cream, a foundation, a lipstick, an eyeshadow, a blusher, an eyeliner, a mascara, a lip care product or a nail care product.

8. A compound with formula (I'):
R_{F}-(CH₂)ₙ-X-[C₃H₅(OH)]-Y-(CH₂)ₘ-R'_{F} (I')
where
C₃H₅(OH) represents the structures: 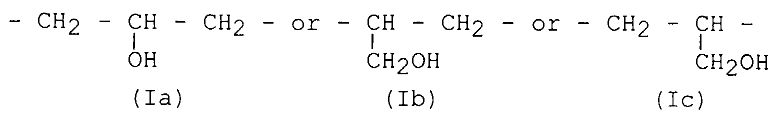
R_{F} and R'_{F}, which may be identical or different, represent a perfluorinated linear or branched C₄-C₂₀ alkyl radical or a mixture of perfluorinated linear or branched C₄-C₂₀ alkyl radicals;
m and n, which may be identical or different, are 0, 1, 2, 3 or 4, and X represents O and Y represents S or X represents S and Y represents O.
